# EUROPEAN PATENT SPECIFICATION

(11) **EP 2 654 807 B1**
(45) Date of publication and mention of the grant of the patent: **19.04.2017**
(21) Application number: 11805517.7
(22) Date of filing: 22.12.2011
(51) Int. Cl.: A61L 2/10, A61L 2/24, B01L 1/04

(54) **CLEAN ROOM DEVICE WITH MOVABLE ELECTROMAGNETIC RADIATION SOURCE**
REINRAUMVORRICHTUNG MIT BEWEGLICHER ELEKTROMAGNETISCHER STRAHLUNGSQUELLE
DISPOSITIF DE SALLE BLANCHE PRÉSENTANT UNE SOURCE DE RAYONNEMENT ÉLECTROMAGNÉTIQUE MOBILE

(30) Priority: 22.12.2010 EP 10196674; 18.04.2011 EP 11162850
(43) Date of publication of application: 30.10.2013
(73) Proprietor: Ortner Cleanroom Engineering GmbH, 9500 Villach (AT)
(72) Inventor: LIEBMINGER, Stefan, A-8041 Graz (AT); STAMPF, Roland, A-9500 Villach (AT)
(74) Representative: Haeusler, Rüdiger Hans-Jörg
(86) International application number: PCT/EP2011/073885
(87) International publication number: WO 2012/085250

(56) References cited:
- WO-A1-99/49823
- WO-A1-2005/054217
- WO-A1-2010/097333
- DE-A1-102009 022 344
- FR-A1- 2 924 445
- US-A- 4 304 224
- US-A- 5 634 711
- US-A- 5 920 075
- US-A1- 2004 219 057
- US-A1- 2005 079 096

## Description

The invention relates to a decontamination system.

Furthermore, the invention relates to a method of using a clean room device.

A clean room is an environment, typically used in manufacturing or scientific research for instance in the context of semiconductor or pharmaceutical or medical technology, that has a low level of environmental pollutants such as dust, airborne microbes, aerosol particles and chemical vapors. More accurately, a clean room has a controlled level of contamination that is specified by the number of particles per cubic meter at a specified particle size.

A clean room suit is an overall garment worn by a user in a clean room. One common type is an all-in-one coverall worn by semiconductor and nano-technology line production workers, technicians, and process engineers, as well as people in similar roles creating sterile products for the medical device industry.

However, entry into or exit out of a clean room may be cumbersome for a user and may involve challenges related to a reliable decontamination.

WO 2010/097333 A1 discloses a clean room suit and a clean room lock. The clean room suit is intended to be used for clothing a person in a clean room and comprises a fabric configured to cover at least a part of a body of the person, and a clean room lock adapter arranged on and/or in the fabric and being configured for coupling to a respective clean room suit adapter of the clean room lock. The clean room lock may comprise fixedly mounted ultraviolet radiation sources configured for irradiating the clean room suit with ultraviolet radiation.

It is an object of the invention provide an efficient mechanism according to which a clean room is accessible.

In order to achieve the object defined above, a decontamination system, and a method of using a clean room device according to the independent claims are provided.

According to an exemplary embodiment of the invention, a decontamination system is provided, wherein the decontamination system comprises a clean room arrangement which comprises one of the group consisting of a clean room and a clean room lock, and a clean room device being arranged at the clean room arrangement and comprising a movably configured (for instance movably mounted or configured with a dimension and/or weight that it can be handled and displaced manually by a user) electromagnetic radiation source for irradiating parts of a clean room equipment with electromagnetic radiation for decontaminating a surface to be treated of the clean room equipment.

According to another exemplary embodiment of the invention, a clean room device having the above mentioned features is used for irradiating parts of a clean room equipment located at a clean room arrangement for decontaminating a surface to be treated of the clean room equipment, wherein the clean room arrangement comprises a clean room lock, and wherein the clean room equipment is configured as clean room clothing to be worn by a person in the clean room lock.

More specifically, a decontamination system is provided which comprises a clean room lock and a hand-held device (or a portable device) which comprises as integral part thereof an electromagnetic radiation source, particularly one or more light emitting diodes, for irradiating parts of a clean room suit to be worn by a person at a clean room lock with electromagnetic radiation and being arranged at the clean room lock. Beyond this, a method of using a hand-held device having the above mentioned features for irradiating parts of a clean room suit worn by a person at a clean room lock is provided.

The term "electromagnetic radiation", as used herein, refers to a form of energy exhibiting wave like behavior and having both electric and magnetic field components, which oscillate in phase perpendicular to each other as well as perpendicular to the direction of energy propagation. Electromagnetic radiation is classified according to the frequency of its wave. In order of increasing frequency and decreasing wavelength, electromagnetic radiation includes radio waves, microwaves, infrared radiation, visible light, ultraviolet radiation, X-rays, and gamma rays. Hence, the term "electromagnetic radiation", as used herein, denotes photons, particularly in the visible range and/or in the ultraviolet range.

The term "electromagnetic radiation source", as used herein, denotes any physical entity capable of generating photons, particularly in the visible range and/or in the ultraviolet range. Examples are light-emitting diodes, plasma lamps, bulbs, etc.

The term "movable electromagnetic radiation source", as used herein, denotes a physical property of the electromagnetic radiation source that it has a provision allowing a spatial position thereof to be freely adjusted in accordance with the requirements of a certain decontamination procedure. In one embodiment, movability is achieved by implementing the electromagnetic radiation source in a hand-held device which is portable manually by a user. In another embodiment, movability is achieved by providing a mechanical drive mechanism, particularly a motor-based drive mechanism, configured for mechanically moving the electromagnetic radiation source in an automated manner.

The term "surface to be treated", as used herein, denotes any surface that can be colonized by microbial organisms (i.e. contaminants) and to which the one or more photosensitizers of the present invention can be applied. The term includes surfaces made of various materials (e.g., crystalline and amorphous solids, gels, foams, and liquids). The surface may have any properties with respect to rigidity, flexibility, porosity, and the like. In some embodiments, at least two (or more) surfaces (which may be part of the same or of different goods or equipment) are treated concomitantly.

In preferred embodiments, the surface to be treated is part of clean room equipment. The term "clean room equipment", as used herein, refers to any goods that are required for a production process, for purposes of research and development (e.g. in a scientific facility or in a company), etc., in a clean room environment including *inter alia* clean rooms as such, clean room locks, furniture, any types of devices or apparatuses (e.g., pipettes, robots, centrifuges, work benches, computer systems, and the like), laboratory supplies (e.g., tubes, culture dishes, pipette tips, pens, paper, notebooks, packaging containers, and the like), clean room clothing, and any material or body which shall be introduced into a clean room.

The term "clean room", as used herein, denotes an area in which quality of air, temperature, humidity, and particle concentration are usually regulated and monitored in order to protect sensitive equipment from contamination. Air in a clean room may be repeatedly filtered to remove dust particles, germs and/or other impurities. Hence, a clean room may be denoted as a room in which the concentration of air-born particulate matter may be controlled at specific limit to facilitate the manufacture of sterile and highly purified products. In pharmaceutical and medical technology, such particles may to be controlled and monitored and germs may be eliminated which might deteriorate the sterile properties of such a room.

The term "clean room lock", as used herein, denotes a device or chamber permitting the passage of people (and optionally objects) between a clean room ("clean side") and a surrounding ("unclean side"). Such a lock may comprise a small chamber with for instance two airtight doors in series which (in the absence of an emergency case) do not open simultaneously. Hence, a clean room lock can be used to allow passage of persons and objects between a clean environment and a less clean environment. Therefore, within such a lock, cleaning procedures may be performed.

The term "clean room clothing", as used herein, refers to clean room clothing, that is, the protective clothing put on before entering a clean room. Within the present invention, the term "clean room clothing" denotes any type of garment such as, e.g., gloves, face masks, bouffants, surgical caps, head covers, hairnets, full cover hoods, socks, shoe covers, sticky mats, sheets, undergarments, coveralls, jackets, shirts, pants, frocks, isolation gowns, and coats. The design of such protective clothing including materials of fabrication as well as functional properties is well known in the art. In a specific embodiment, the clean room clothing used is provided with one or more antimicrobial agents of inorganic origin, for example gold and/or silver fibers incorporated in the fabrication materials.

Usually, clean room clothing is made of one or more textile fabrics. The term "textile fabric" (or "fabric"), as used herein, particularly denotes any flexible material which can be used as a basis for a garment or a suit. For example, such a fabric may comprise a textile material which, in turn, may comprise a network of natural or artificial fibers such as thread or yarn. A textile fabric may be made by weaving or felting or knitting or crocheting natural or synthetic fibers. Many such fibers are well known in the art and commercially available from numerous suppliers (cf., e.g., Gail, L. and Hortig, H.P. (2004), *supra*).

The term "person" or "user" may particularly denote a human being wishing to enter or leave a clean room. The term "person" should be considered as a human being of an average size, particularly between 1,50 m and 1,90 m in height and between 40 kg and 120 kg of weight. However, also children can be considered as persons in the context of the present application.

The term "photosensitizer", as used herein, refers to any compounds that can be activated by light or, more generally, by radiation and that exhibit, upon such activation, antimicrobial activity. Without the intention of being bound by a particular hypothesis, photosensitizers commonly act by producing, upon radiation (light)-induced activation, reactive chemical species (e.g., singlet oxygen) that exert (non-selective) antimicrobial efficacy. Preferably, the one or more photosensitizers do not exhibit any adverse effects on human health.

The term "antimicrobial efficacy", as used herein, denotes an inhibitory (or antagonistic) effect on the growth of microorganisms. In other words, agents that are capable of at least reducing the growth rate (e.g., bacteriostatic agents with respect to controlling the growth of bacteria) as well as agents that cause toxic effects (e.g., bactericide agents killing bacteria) demonstrate antimicrobial efficacy.

According to an exemplary embodiment of the invention, a clean room device being part of a decontamination system or being used for irradiating parts of a clean room equipment is provided which allows for a spatially well-defined irradiation of specific surface regions of a clean room equipment with photons to thereby activate a photon-induced decontamination mechanism such as a photosensitizer-based mechanism. The latter mechanism is based on a decontaminating effect on a surface of clean room equipment resulting from an interaction between photons in an appropriate wavelength range and the photosensitizer. In certain scenarios in a clean room environment, it may happen that it is difficult to get access to certain positions or surface sections of clean room equipment with the activating photons. By configuring the photon source movable (particularly translatable and/or pivotable), access to even very narrow surface portions of the clean room equipment is enabled.

In the following, further exemplary embodiments of the clean room device being part of a decontamination system or being used for irradiating parts of a clean room equipment will be explained.

Power supply to the electromagnetic radiation source may be performed in a wireless manner (e.g. via a battery) or in a wired manner (e.g. by a cable being electrically coupled to a mains supply or the like). The device may hence be powered via a cable or any other wired connection for electric coupling to a device-external energy source (for instance of a clean room lock), or by means of a power supply unit integrated in the clean room lock (for instance an accumulator).

In an embodiment, the clean room device is configured as a hand-held device comprising as integral part thereof the electromagnetic radiation source, particularly one or more light emitting diodes, for irradiating parts of the clean room equipment configured as a clean room suit, to be worn by a person at a clean room lock with electromagnetic radiation. This provides for a very easy, user-defined mechanism for controlling the irradiation position, angle, distance and/or duration. A hand-held device may have a mass of less than 3 kg, particularly of less than 2 kg, more particularly of less than 1 kg.

In an embodiment, the hand-held device comprises a grip grippable by a user and a radiation head connected to the grip, the electromagnetic radiation source being arranged at the radiation head. Hence, the user may operate the mechanism simply with her or his hands.

In an embodiment, the hand-held device comprises a handling guidance configured for guiding a user to handle the hand-held device in accordance with a predefined sequence of instructions indicative of a defined decontamination procedure. Such a handling guidance may provide the user with instructions as to how a decontamination procedure in a clean room should be performed, for instance a sequence of decontaminations steps. The handling guidance may be, for instance, a display device in combination with a control unit (such as a CPU) providing display data for displaying instructions concerning the decontamination procedure on the display unit (such as an LCD display). Hence, correctness and reproducibility of the decontamination procedure may be improved. Consequently, compliance with validity requirements, as may be defined by an official authority, may be guaranteed.

In an embodiment, the clean room device is configured as a scanner, particularly a robotic scanner, wherein the scanner comprises a scanner head comprising as integral part thereof the electromagnetic radiation source, particularly one or more light emitting diodes, and a drive unit configured for driving the scanner head to move along a predefined trajectory for scanning the clean room equipment so as to irradiate parts of the clean room equipment with electromagnetic radiation. Thus, an automatic scan procedure may be implemented which allows for a reliable and reproducible irradiation process.

Upon using a scanner, a body contour or surface contour of the clean room equipment to be decontaminated may be scanned, wherein a minimum distance between light source and surface of the clean room equipment is not exceeded during the scanning procedure as a result of a guided motion.

In order to increase the decontaminating precision during scanning (or during a use of a hand-held device by a user), a provision may be taken instructing a user to assume a body position which is appropriate for a complete irradiation of the clean room equipment configured as a clean room suit in this embodiment. For instance, one or more markers may be foreseen indicating a preferred position of the feet of the user during use of the clean room device. For instance, one or more grips may be foreseen indicating a preferred position of the hands of the user during use of the clean room device.

In an embodiment, the scanner is configured for moving the scanner head along the predefined trajectory in accordance with a predefined sequence of instructions indicative of a defined decontamination procedure. Therefore, completeness of the decontamination process may be rendered independently of the skills and the reliability of a user.

In an embodiment, the electromagnetic radiation source is configured for emitting the electromagnetic radiation with a wavelength in a range configured for activating one or more photosensitizers, wherein the one or more photosensitizers are reactive dyes, and particularly reactive dyes being selected from the group consisting of methylene blue, methyl methylene blue, dimethylmethylene blue, new methylene blue, toluidine blue, Rose Bengal, acridine orange, and hypericin. In most preferred embodiments, the one or more photosensitizers are selected from the group consisting of methyl methylene blue, dimethylmethylene blue, and new methylene blue.

In an embodiment, the electromagnetic radiation source is configured for emitting the electromagnetic radiation being emitted has a wavelength in a range between about 400 nm and about 800 nm, particularly in a range between about 580 nm and about 650 nm, more particularly in a range between about 610 nm and about 640 nm, even more particularly exclusively in a range between about 610 nm and about 640 nm (wherein "exclusively" means that no radiation at all is emitted outside of the respective range of wavelengths). The range between about 610 nm and about 640 nm has turned out to be particularly appropriate for the activation of most preferred photosensitizers.

It is also possible that the electromagnetic radiation source is configured for emitting electromagnetic radiation having a wavelength in two different, non-consecutive ranges, each of which being configured for activating one of two different photosensitizers. By using multiple different photosensitizers, synergetic effects in decontaminating may be achieved.

In an embodiment, the clean room device comprises a clean room interface (such as a plug) configured for providing for a coupling, particularly at least one of a power supply coupling for supplying the clean room device with power and a communicative coupling for transmitting communication data, between the electromagnetic radiation source and a clean room arrangement in which the clean room device is to be located. Such a clean room interface may be wired or wireless. It may serve for supply of power from the clean room arrangement to the clean room device (for instance wired by an ohmic transport of current or wireless by a capacitive and/or an inductive supply of electric power). Additionally or alternatively, it may serve for transmission of data (such as decontamination instructions) from the clean room arrangement to the clean room device (for instance wired via a cable or wireless by Bluetooth or the like), or *vice versa.* The clean room device may be selectively attachable or detachable for enabling or disabling cooperation via the interface.

In an embodiment, the clean room device comprises a control unit (such as a central processing unit or a microprocessor) configured for driving the electromagnetic radiation source for emitting the electromagnetic radiation in accordance with a predefined decontaminating procedure. Data defining the decontaminating procedure may be stored in a database, for instance in a mass storage device to be accessed and executed by the control unit.

In an embodiment, the clean room equipment is configured as a clean room suit to be worn by a person in a clean room lock. By the movable light source, even parts of the clean room suit being difficult to access (such as kinked surface areas of fabric) may be accessed easily.

In an embodiment, the clean room equipment is provided with one or more photosensitizers being activatable by the electromagnetic radiation, wherein the one or more photosensitizers are particularly reactive dyes, and more particularly reactive dyes being selected from the group consisting of methylene blue, methyl methylene blue, dimethylmethylene blue, new methylene blue, toluidine blue, Rose Bengal, acridine orange, and hypericin. In an embodiment, the one or more photosensitizers are capable of exhibiting antimicrobial efficacy upon activation.

The wavelength of the electromagnetic radiation that is used when performing the method of to the present invention is selected (that is, specifically configured or adapted) depending on the one or more photosensitizers provided on the surface to be treated, that is, the electromagnetic radiation (i.e. light) required for activation of the photosensitizers in order to exert their antimicrobial efficacy. The skilled person is well aware how to select an appropriate wavelength depending on the type of photosensitizer(s) used. The type of electromagnetic radiation source emitting radiation having the appropriate wavelength for a given scenario is selected accordingly.

Suitable electromagnetic radiation source to be used in the present invention include *inter alia* laser, mercury lamps, black light lamps, white light lamps, infrared lamps, and light emitting diodes (LEDs), with the latter one being particularly preferred. All these radiation sources are well established in the art.

Within the present invention, one or more electromagnetic radiation sources may be used, which may be of the same type (e.g., at least two LEDs) or of different types (e.g., at least one LED and at least one laser), again depending on the type of photosensitizer(s) provided on the surface to be treated. The one or more electromagnetic radiation sources are arranged in a manner suitable to emit radiation towards the surface to be treated, for example, an arrangement opposite of the surface to be treated. The distance between the one or more electromagnetic radiation sources and the surface to be treated may be in a range between 1 cm and 10 m or in a range between 5 cm and 5 m, and preferably in a range between 10 cm and 4 m or in a range between 20 cm and 3 m. In particularly preferred embodiments, the distance may be in a range between 30 cm and 2.5 m or in a range between 50 cm and 2 m.

In the following, further exemplary embodiments of the decontamination system will be explained. However, these embodiments also apply to the method of using.

The clean room arrangement comprises one of the group consisting of a clean room, particularly an isolator or a digestorium, and a clean room lock.

An isolator can be denoted as a type of clean air device that creates an almost complete separation between a product and its production equipment, technical personnel, and surrounding work environment. Isolators are generally used in applications requiring a high degree of protection from external elements or contaminants, and they can also serve as alternatives to sophisticated clean rooms. Isolators typically feature built-in air filtration systems capable of significantly lowering a particle count in an enclosed area, and their relatively compact size makes it easier to clean their interiors with gas or vapor sterilizing agents. A light positive air pressure may be maintained to prevent the entrance of contaminants from any openings, while worker activities may be conducted through sealed glove assemblies.

A digestorium or fume hood or fume cupboard can be denoted as a type of local ventilation device that is designed to limit exposure to hazardous or noxious fumes, vapors or dusts. A fume hood is typically a large piece of equipment enclosing five sides of a work area, the bottom of which is most commonly located at a standing work height.

In an embodiment, the decontamination system comprises at least one further electromagnetic radiation source being fixedly mounted at the clean room arrangement to emit electromagnetic radiation onto a fixed position of the clean room arrangement. The one or more further electromagnetic radiation sources may be static and therefore mountable with low effort to irradiate a large portion of the surface to be treated. Remaining surface portions being not easily accessible for static light sources may then be selectively treated by the one or more movable electromagnetic radiation sources.

In an embodiment, the at least one further electromagnetic radiation source is configured as the electromagnetic radiation source mentioned above, i.e. may be particularly configured for activating a decontamination procedure by impinging on a surface of the clean room equipment.

In an embodiment, the at least one further electromagnetic radiation source is integrated in a wall, a floor, and/or a ceiling of the clean room arrangement.

In the following, further exemplary embodiments of the method of using will be explained. However, these embodiments also apply to the decontamination system.

In an embodiment, the clean room device is used by a person transmitting between an interior and an exterior of a clean room, i.e. in a clean room lock.

In an embodiment, the clean room device is used for the microbial decontamination of a surface of the clean room equipment to be treated.

Embodiments of the invention may be applied to the decontamination of any type of surface by means of a manually controlled or machine controlled light source. The light emission intensity of the light source should be chosen so that a desired extent of decontamination is achieved.

The motion trajectory of the clean room device may be unalterably controlled or may be controllable in accordance with a given or determined or optimized motion sequence. Corresponding control data may be stored in the device or in the clean room arrangement and may be used as a basis for a fixed control or as a basis for an instruction to a user. A corresponding reproducible decontamination scan may meet criteria of validity.

The aspects defined above and further aspects of the invention are apparent from the examples of embodiment to be described hereinafter and are explained with reference to these examples of embodiment.

The invention will be described in more detail hereinafter with reference to examples of embodiment but to which the invention is not limited.
Figure 1 illustrates an embodiment of the invention with a clean room lock and a portable hand-held device installed therein by which a user wearing a clean room suit is enabled to irradiate selective portions thereof with UV light to initiate a decontamination procedure.
Figure 2 illustrates a clean room lock with translatable decontamination light source scanners according to an embodiment of the invention.
Figure 3 illustrates a clean room lock with a portable decontamination light source according to an embodiment of the invention.
Figure 4 illustrates a clean room lock with a pivotable decontamination light source scanner according to an embodiment of the invention.
Figure 5 illustrates a hand-held decontamination light source according to an embodiment of the invention.
Figure 6 illustrates photosensitizers according to embodiments of the present invention.
Figure 7 illustrates photosensitizers according to embodiments of the present invention.
Figure 8 illustrates an analysis of the antimicrobial efficacy of methylene blue attached to textile fabrics (contact assay) according to an embodiment of the present invention.

The illustration in the drawing is schematically. In different drawings, similar or identical elements are provided with the same reference signs.

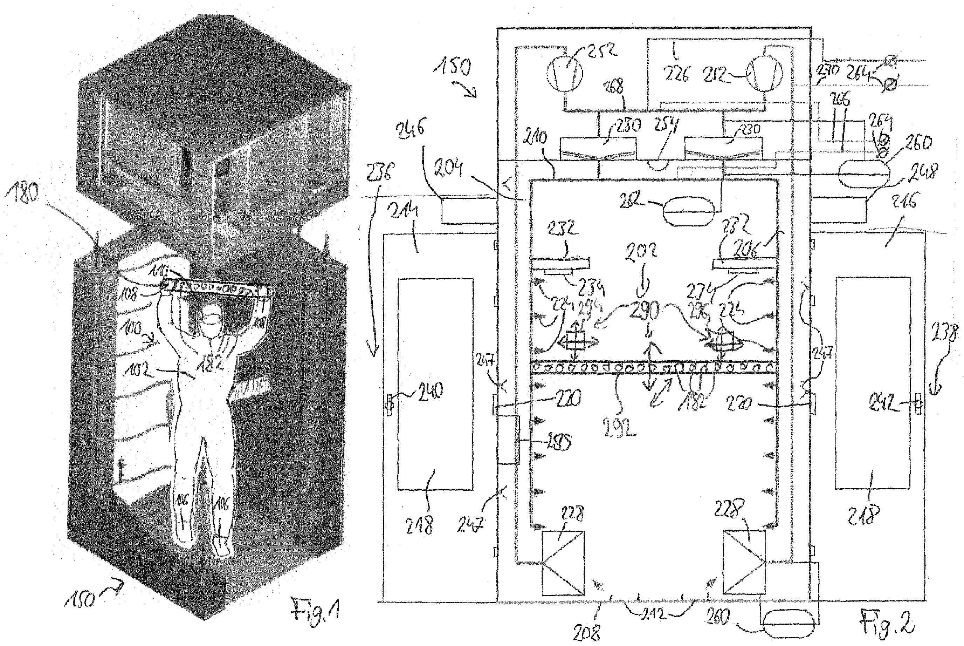

**Fig. 1** illustrates a person wearing a clean room suit 100 and standing within a clean room lock 150 according to an exemplary embodiment of the invention.

The clean room suit 100 comprises an overall-like fabric 102 covering a major portion of a body of the person, and therefore being configured as an overall clean room suit.

More specifically, a hand-held device 180 is provided which comprises as integral part thereof multiple light emitting diodes 182 for irradiating parts of the clean room suit 100 worn by the person at the clean room lock 150 with visible light of an appropriate wavelength. In the shown embodiment, the person holds the rod-like hand-held device 180 with both hands. However, since hand-held device 180 is portable, the user may even hold and operate the hand-held device 180 with one hand only. For decontaminating the clean room suit 100, the person may apply a photosensitizer to the surface of the clean room suit 100. The person may then activate the light emitting diodes 182 for irradiating light onto the surface of the clean room suit 100. By manually moving the hand-held device 180, the person may specifically irradiate problematic surface portions such as kinks of the fabric 102 to ensure a decontamination of the whole surface to be treated. By an interaction of the photosensitizer and the light, germs on the fabric 102 may be deactivated.

As can be taken from Fig. 1, only the hands, the feet and an eye portion of the person are not covered by the fabric 102. However, the feet are covered with clean room shoes 106 being sealedly connected with the fabric 102. Furthermore, the hands of the person are covered by gloves 108 being sealedly connected with the fabric 102 and the eyes are covered with optional protection goggles 110.

Further features which may be implemented in the clean room lock 150 of Fig. 1 will be described in more detail referring to **Fig. 2****.**

The clean room lock 150 of Fig. 2 encloses a person cabin 202 having sidewalls 204, 206, a bottom wall 208 and a ceiling wall 210. The dimension of the cabin 204 is so that a standing adult person fits properly into the cabin 202. Markers 212 (for instance a target circle or individual foot markers) are provided on the transparent bottom wall 210 to indicate a position where the person should stand in the cabin 202. When the feet of the person are within the marker portions 212, the person stands in the cabin 202 with slightly straggled legs, for instance with an angle of 15° between the legs.

The material of the bottom wall 208 is transparent for optical radiation, for instance may be a quartz glass plate with an aluminium reflector. Although not shown in Fig. 2, further light sources may be arranged below the transparent bottom 208 so as to irradiate a surface of the clean room suit 100, particularly a sole of the clean room shoes 106, with electromagnetic radiation for decontamination purposes. For decontaminating the clean room shoes 106, the person may apply a photosensitizer to the surface of the clean room shoes 106, and light from the light sources in the bottom will disinfect the clean room shoes 106. Also close to the sidewalls 204, 206, a number of light sources 247 are arranged for laterally irradiating the cabin 202 with optical radiation for supporting the above described decontamination process.

Sidewall 204 comprises a first door 214, whereas sidewall 206 comprises a second door 216. The doors 214, 216 each have a glass element 218. The doors 214, 216 also have a corresponding electrical closure mechanism 220. Grips of the doors 214, 216 are indicated with reference numerals 240 and 242, respectively.

A gas jet system is provided which comprises nozzle rows each having eight nozzles 224 for blowing a gas towards the cabin 202, thereby also directing a gas flow around an exterior of the clean room suit 100. Via optional conduits 266, a chemical decontamination medium such as DEHS (Diethylhexylsebacate) may be supplied in the cabin 202. The chemical decontamination agent may be supplied to the cabin 202 via the ceiling 210 and/or via the side walls 204, 206.

A suction system may provide an under pressure close to the bottom wall 208 of the cabin 202 and may suck off gas and/or liquid from the cabin 202 via pre-filters 228 and filters for suspended particles 230. Ventilators 252 may provide a corresponding pump performance. After filtering and hence cleaning, the filtered gas can be supplied back to the cabin 202.

Bow-type handles 232 are arranged in the cabin 202 above the person so that arms of the person are raised above a head of a person when the hands of the person grip the handles 232. The handles 232 comprise touch sensors 234, wherein the touch sensors 234 generate a signal triggering start of a predefined cleaning procedure upon detecting that the hands of the persons properly grip the handle 232, and therefore also touch the touch sensors 234. Other kinds of sensors are of course possible.

A clean side at which a clean room is directly connected is indicated with reference numeral 236, whereas an unclean side (a surrounding of the clean room) is indicated with reference numeral 238.

A user interface 246 at the clean side 236 allows a user to control an operation mode of the cleaning procedure, allows for a control of the photosensitizer-based decontamination and may house a central control unit for controlling the system 100, 150. A further user interface 248 is provided as well at the unclean side 238. A display panel 250 which, inter alia, includes a start button and an emergency stop and exit button is shown in the cabin 202 as well.

A motion sensor 254 is arranged at the ceiling wall 210 and may detect when the person moves after starting of a cleaning performance in the cabin 202 and can take corresponding measures, for instance may terminate the cleaning procedure or may repeat the cleaning procedure.

Optional PDAH units 260 can be seen at various positions. A PDAL unit 262 is shown as well.

Control valves 264 can be controlled individually by the control unit 246 or by a human operator, for instance for activating or deactivating a DEHS injection (which may be measured as well) via fluid conduits 266.

Air delivery can be achieved via a conduit 226, whereas exhaust air can be conducted via a conduit 270.

A scanner 290 is provided as well for supporting the decontamination procedure and comprises four rod-like scanner heads, i.e. a front scanner head 292 extending over the whole width of the accommodation chamber, a first side scanner head 294 extending over the whole length of the accommodation chamber (perpendicular to the paper plane of Fig. 2), a second side scanner head 296 extending over the whole length of the accommodation chamber (perpendicular to the paper plane of Fig. 2) parallel to the first side scanner head 294, and a rear scanner head (not shown since it is located behind front scanner head 292) extending over the whole width of the accommodation chamber as well. Each scanner head can be moved along two dimensions, as indicated by arrows in Fig. 2. Each scanner head 292, 294, 296 comprises as integral part thereof light emitting diodes , particularly one or more light emitting diodes 182. A drive motor (not shown) is configured for driving each of the scanner heads 292, 294, 296 to move along a predefined trajectory for scanning clean room equipment (not shown) to be covered with photosensitizer material for decontamination so as to irradiate parts of the clean room equipment with the light. A control unit may control the motion trajectories to be scanned by the scanner heads 292, 294, 296.

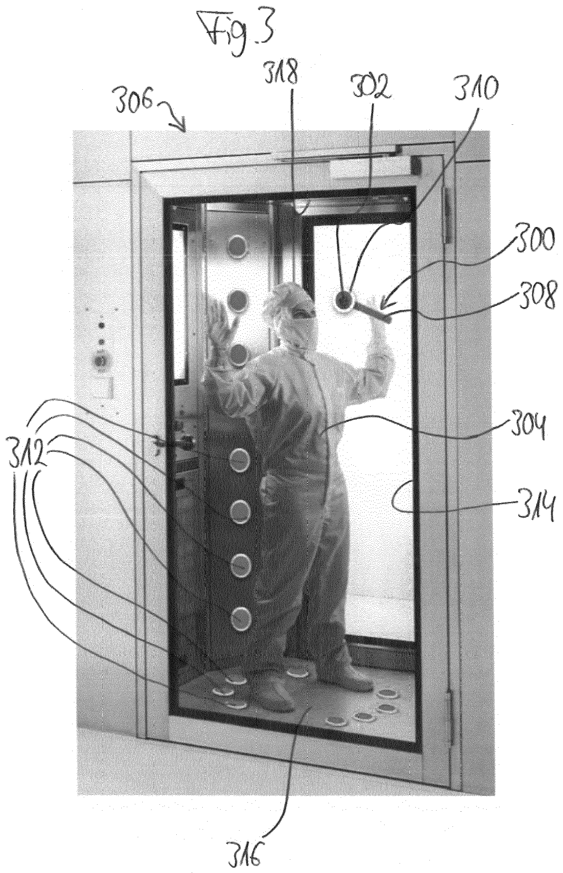

**Fig. 3** illustrates a clean room lock 306 according to an embodiment of the present invention.

Shown is an exemplary embodiment of a clean room lock 306 for the decontamination of clean room clothing 304 according to the present invention. A person transmitting between an interior and an exterior of a clean room is wearing a clean room suit 304 provided with one or more photosensitizers being activatable by electromagnetic radiation. The person is standing in the clean room lock 306, which comprises multiple electromagnetic radiation sources 302, 312 (such as LEDs; shown as spots) being arranged to emit electromagnetic radiation on the clean room suit 304 for activating the photosensitizers. Most of the electromagnetic radiation sources 312 are integrated in the walls 314, the floor 316, and the ceiling 318 of the clean room lock 306. One radiation source 302 is integral part of a hand-held device 300 for irradiating any parts of the clean room suit 304 that are difficult to be accessed.

More specifically, the hand-held device 300 comprises as integral part thereof the electromagnetic radiation source 302 having one or more light emitting diodes, for irradiating parts of the clean room suit 304 to be worn by a person in the clean room lock 306 with electromagnetic radiation, particularly with visible light. The hand-held device 300 comprises a grip 308 grippable by a hand of a human user and a radiation head 310 connected to the grip 308, the electromagnetic radiation source 302 being arranged at the radiation head 310. The electromagnetic radiation source 302 is configured for emitting the electromagnetic radiation with a wavelength in a range configured for activating one or more photosensitizers, wherein the one or more photosensitizers are reactive dyes, and particularly reactive dyes being selected from the group consisting of methylene blue, methyl methylene blue, dimethylmethylene blue, new methylene blue, toluidine blue, Rose Bengal, acridine orange, and hypericin. More particularly, the electromagnetic radiation source is configured for emitting the electromagnetic radiation being emitted has a wavelength in a range between 400 nm and 800 nm, particularly in a range between 580 nm and 650 nm, more particularly in a range between 610 nm and 640 nm, even more particularly exclusively in a range between 610 nm and 640 nm.

Although not shown in Fig. 3, the hand-held device 300 may have a control interface (such as a wired or a wireless communication interface) communicatively coupled to a control unit (not shown) of the clean room lock 306. This control unit may send a control signal to the control interface for enabling or triggering emission of electromagnetic radiation by the hand-held device 300. Thus, the hand-held device 300 may operate under control of the clean room lock 306. Hence, it may be ensured that the hand-held device 300 emits electromagnetic radiation only when the decontamination procedure in the clean room lock 306 has started or has been approved. Thus, misuse may be prevented. Furthermore, the hand-held device 300 may comprise a button to be actuated by a user so as to start an emission of electromagnetic radiation of the hand-held device 300 by a user. Hence, energy consumption may be kept as small as possible.

In combination, the clean room suit 304 (worn by the person in the clean room lock 306) and the hand-held device 300 for irradiating parts of the clean room suit 304 for decontamination form a decontamination kit.

The clean room suit 304 is provided with one or more photosensitizers being activatable by the electromagnetic radiation, wherein the one or more photosensitizers are particularly reactive dyes, and more particularly reactive dyes being selected from the group consisting of methylene blue, methyl methylene blue, dimethylmethylene blue, new methylene blue, toluidine blue, Rose Bengal, acridine orange, and hypericin. The one or more photosensitizers are capable of exhibiting antimicrobial efficacy upon activation.

A decontamination system is formed by the clean room lock 306 and the hand-held device 300 which is located in the clean room lock 306.

The hand-held device 300 can be used for irradiating parts of a clean room suit 304 worn by the person at the clean room lock 306. Particularly, the hand-held device 300 is used by a person transmitting between an interior and an exterior of a clean room for the microbial decontamination of a surface of the clean room suit 304 to be treated.

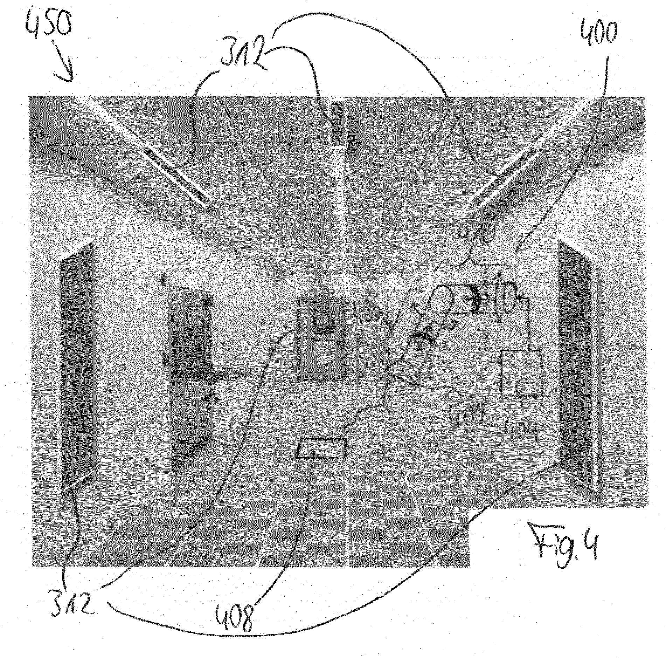

**Fig. 4** illustrates a clean room 450 adapted for practicing an embodiment of the present invention.

Shown is an exemplary embodiment of a clean room 450 that is adapted for practicing the present invention. The clean room is equipped with six electromagnetic radiation sources 312: three being arranged at the ceiling, two at the left and right side walls, and one surrounding the entrance in the back. The stationary mounted radiation sources 312 may be used for permanently irradiating the interior of the clean room 450.

Furthermore, a clean room device 400 is provided and configured as a robotic scanner having a scanner head 402 comprising as integral part thereof a movably mounted electromagnetic radiation source 302. An electric motor acts as a drive unit 404 for mechanically driving the scanner head 402 to move along a predefined trajectory for scanning a surface of a clean room suit worn by a user (both not shown) standing on a marking 408 so as to irradiate parts of the clean room suit with light in an appropriate wavelength range for activating a photosensitizer applied to the clean room suit for decontamination.

More specifically, the scanner is configured for moving the scanner head 402 along the predefined trajectory in accordance with a predefined sequence of instructions indicative of a defined decontamination procedure. This sequence can be stored in a storage device of the drive unit 404.

The clean room device 400 has a first robotic arm 410 (being pivotable and being changeable in length by a telescopic unit) and has a second robotic arm 420 (being pivotable and being changeable in length by a telescopic unit), wherein both robotic arms 410, 420 are movable under the control of the drive unit 404.

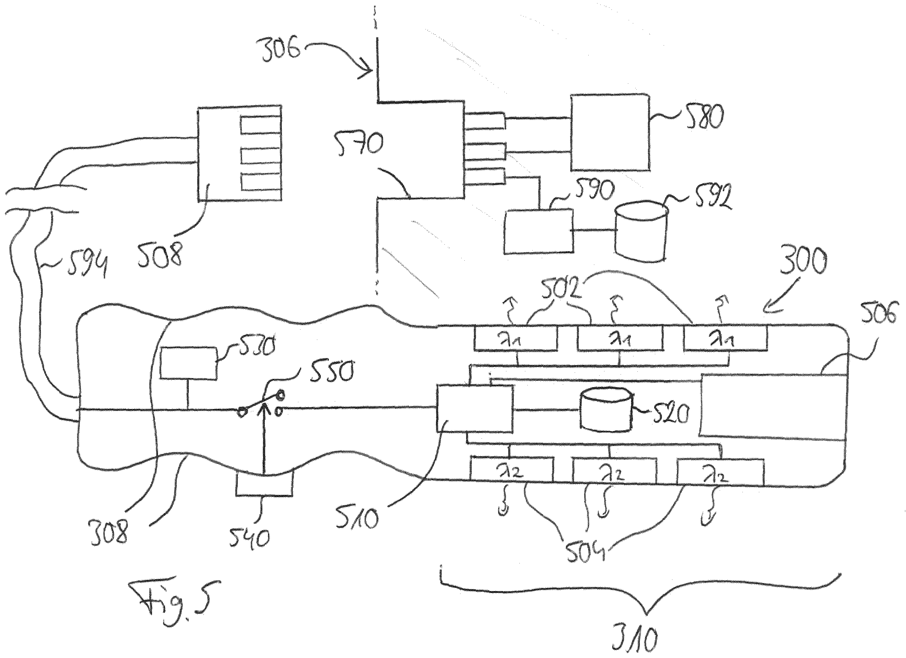

**Fig. 5** illustrates a hand-held decontamination light source 300 according to an embodiment of the invention.

The hand-held device 300 comprises as integral part thereof six light emitting diodes 502, 504 (three LEDs 502 emitting light in a first wavelength range, and three LEDs 504 emitting light in a second wavelength range), for irradiating parts of a clean room equipment with light. The hand-held device 300 comprises a grip 308 grippable by a user and a radiation head 310 connected to the grip 308. The six light emitting diodes 502, 504 are arranged at the radiation head 310.

The hand-held device 300 comprises a display device 506 (such as a LCD) as a handling guidance for guiding a user to handle the hand-held device 300 in accordance with a predefined sequence of instructions indicative of a defined decontamination procedure. In other words, instructions may be displayed on display 506 instructing a user as to how to use the hand-held device 300. A control unit 510 can access a database 520 or storage unit to be provided with data to be displayed by display device 506. Electric power for powering the light emitting diodes 502, 504, the display device 506, and the database 520 can be provided by a rechargeable accumulator 530.

A user may operate a button 540 to operate a switch 550 to start a decontamination procedure during which the accumulator 530 supplies to components of the hand-held device 300 with electric power and during which the hand-held device 300 is connected to a clean room lock 306 via a cable 594. The connection is provided by plugging a plug 508 in a socket 570. Upon connecting the hand-held device 300 to the clean room lock 306, the clean room lock 306 may supply electric power for charging the accumulator 530 from power supply unit 580. Furthermore, upon connecting the hand-held device 300 to the clean room lock 306, the clean room lock 306 may supply instruction data from a control unit 590 (or a database 592 connected thereto) to control unit 510.

In an embodiment of the present invention, any of the claimed apparatuses may be used in conjunction with a method for controlling microbial growth on a surface to be treated, the method comprising: exposing the surface to be treated to electromagnetic radiation, the electromagnetic radiation being emitted from one or more electromagnetic radiation sources towards the surface, wherein the surface is provided with one or more photosensitizers being activatable by electromagnetic radiation, the electromagnetic radiation emitted from the one or more electromagnetic radiation sources has a wavelength in a range configured for activating the one or more photosensitizers, and the one or more photosensitizers exhibit antimicrobial efficacy upon activation by electromagnetic radiation.

Embodiments of the present invention are based on the unexpected finding that the provision of a surface to be decontaminated with one or more photosensitizers being activatable by electromagnetic radiation and exposure of the surface to electromagnetic radiation of appropriate wavelength for activating the photosensitizers represents a successful tool for (biologically) controlling microbial growth on such surface, in particular as the use of harmful UV radiation is typically avoided. This, in turn, enables for a reliable and efficient decontamination of such surfaces even in extreme environments (e.g., in clean rooms or on clean room clothing) or in clinical settings, frequently requiring the inhibition and/or killing of human pathogens, without the risk that the contaminants acquire resistance against the antimicrobial agents employed. The latter is particularly accomplished by the non-selective mode of action of the photosensitizers being employed. Typically, upon radiation-induced activation such photosensitizers produce reactive chemical species (e.g., singlet oxygen) exerting antimicrobial efficacy.

The methods and compositions described herein also enable a reliable and efficient decontamination of clean room clothing, thus facilitating clean room operations by avoiding the otherwise necessary change of the protective clothing at each and every entry in or leave of a clean room.

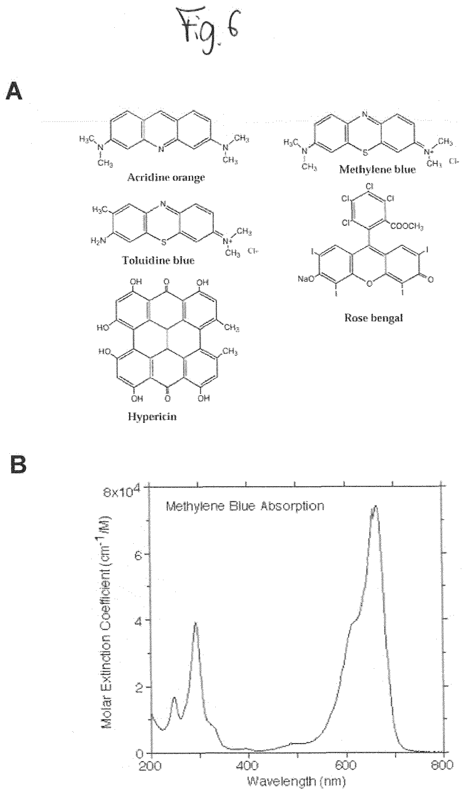

**Fig. 6** illustrates photosensitizers according to embodiments of the present invention. **(A)** Schematic representation of the chemical structures of five exemplary reactive dyes that can be employed as photosensitizers according to the invention. **(B)** Spectral analysis of methylene blue revealing two absorption maxima at 610 nm and 664 nm, respectively.

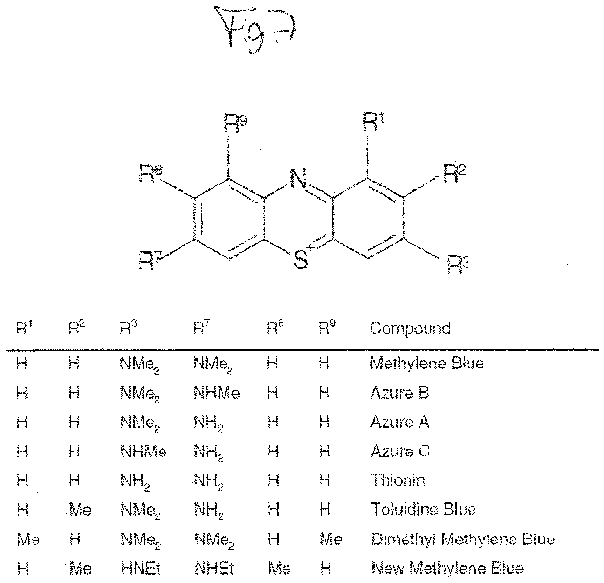

**Fig. 7** illustrates photosensitizers according to embodiments of the present invention. Schematic representation of the chemical structures of methylene blue and seven exemplary derivatives thereof (including toluidine blue) that can be employed as photosensitizers according to the invention (adapted from Wainwright, M. and Amaral, L. (2005) Trop. Med. Int. Health 10, 501-511). Another exemplary derivative, methyl methylene blue, has an identical structure as methylene blue except for a methyl group at substituent R¹.

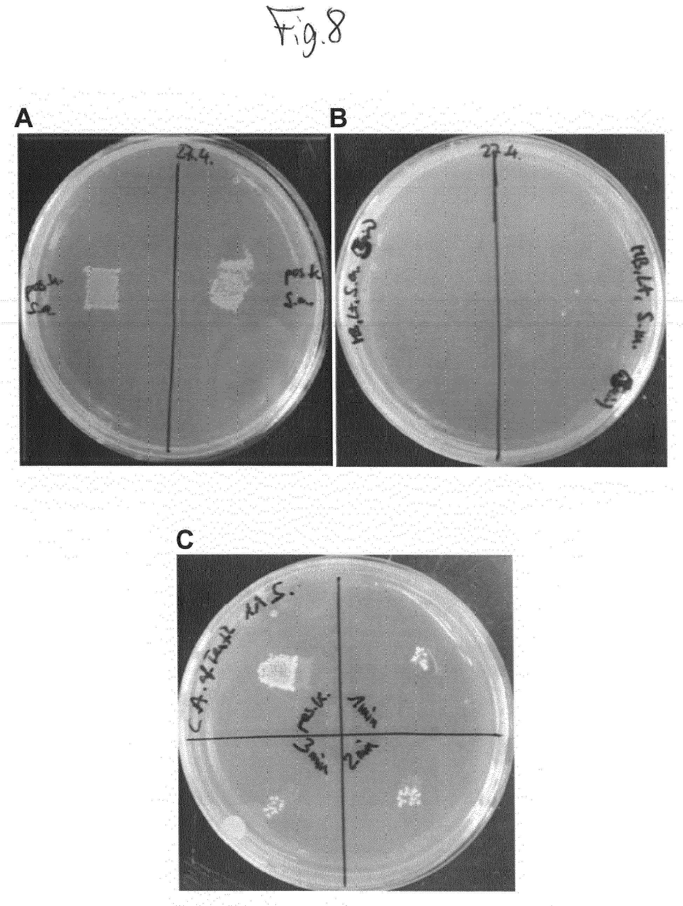

**Fig. 8** illustrates an analysis of the antimicrobial efficacy of methylene blue attached to textile fabrics (contact assay) according to an embodiment of the present invention.

Pieces of a textile fabric (ION-NOSTAT VI.2; Dastex Reinraumzubehör, Muggensturm, Germany) having a defined size (1 x 1 cm) were immersed for 3 h in an aqueous solution of methylene blue (100 µg/ml). After complete drying of the textile pieces, 3 µl of a growing cell culture (titer: 7 x 10⁷ cfu/ml) of *Staphylococcus aureus, Stenotrophomonas maltophilia,* and *Candida albicans,* respectively, were added to the surface of the textile pieces. The textile pieces were contacted with agar plates; and the plates were incubated at 37°C for 24 h. **(A)** Control plate; contact sampling of textile pieces without further treatment: *S. aureus* (left), *S*. *maltophilia* (right). **(B)** Test plate; contact sampling of textile pieces after irradiation for 3 min with a laser (15 mW) at a wavelength of 532 nm: *S. aureus* (left), *S*. *maltophilia* (right). **(C)** Corresponding analysis for *C*. *albicans;* shown are (clockwise, starting at top left) the control, and the test samples after irradiation for 1 min, 2 min, and 3 min, respectively.

It should be noted that the term "comprising" does not exclude other elements or features and the "a" or "an" does not exclude a plurality. Also elements described in association with different embodiments may be combined.

It should also be noted that reference signs in the claims shall not be construed as limiting the scope of the claims.

## Claims

1. A decontamination system, the decontamination system comprising:
a clean room arrangement (306) which comprises one of the group consisting of a clean room and a clean room lock;
a clean room device (300) arranged at the clean room arrangement (306), the clean room device (300) comprising a movably configured electromagnetic radiation source (302) for irradiating parts of a clean room equipment (304) with electromagnetic radiation for decontaminating a surface to be treated of the clean room equipment (304).

2. The decontamination system according to claim 1, wherein the clean room is an isolator or a digestorium.

3. The decontamination system according to claim 1 or 2, comprising at least one further electromagnetic radiation source (312) being fixedly mounted at the clean room arrangement (306) to emit electromagnetic radiation onto a fixed position of the clean room arrangement (306).

4. The decontamination system according to claim 3, wherein the at least one further electromagnetic radiation source (312) is configured as an electromagnetic radiation source (302) having at least one of the following features (i) and (ii):
(i) the electromagnetic radiation source (302) is configured for emitting the electromagnetic radiation with a wavelength in a range configured for activating one or more photosensitizers, wherein the one or more photosensitizers are reactive dyes, and particularly reactive dyes being selected from the group consisting of methylene blue, methyl methylene blue, dimethylmethylene blue, new methylene blue, toluidine blue, Rose Bengal, acridine orange, and hypericin;
(ii) the electromagnetic radiation source (302) is configured for emitting the electromagnetic radiation being emitted has a wavelength in a range between 400 nm and 800 nm, particularly in a range between 580 nm and 650 nm, more particularly in a range between 610 nm and 640 nm, even more particularly exclusively in a range between 610 nm and 640 nm.

5. The decontamination system according to claim 3 or 4, wherein the at least one further electromagnetic radiation source (312) is integrated in at least one of a wall (314), a floor (316), and a ceiling (318) of the clean room arrangement (306).

6. The decontamination system according to any of claims 1 to 5, wherein the clean room device (300) is configured as a hand-held device (300), the hand-held device (300) comprising as integral part thereof the electromagnetic radiation source (302), particularly one or more light emitting diodes (502, 504), for irradiating parts of the clean room equipment (304) configured as clean room clothing, particularly a clean room suit (304) to be worn by a person at a clean room lock (306), with electromagnetic radiation.

7. The decontamination system according to claim 6, wherein the hand-held device (300) comprises a grip (308) grippable by a user and a radiation head (310) connected to the grip (308), the electromagnetic radiation source (302) being arranged at the radiation head (310).

8. The decontamination system according to any of claims 1 to 5, wherein the clean room device (400) is configured as a scanner (400), particularly a robotic scanner (400), the scanner (400) comprising:
a scanner head (402) comprising as integral part thereof the electromagnetic radiation source (302), particularly one or more light emitting diodes (502, 504);
a drive unit (404) configured for driving the scanner head (402) to move along a predefined trajectory for scanning the clean room equipment (304) so as to irradiate parts of the clean room equipment (304) with electromagnetic radiation.

9. The decontamination system according to any of claims 1 to 8, wherein the clean room device (300) comprises a clean room interface (508) configured for providing for a coupling, particularly at least one of a power supply coupling for supplying the clean room device (300) with power and a communicative coupling for transmitting communication data, between the electromagnetic radiation source (302) and a clean room arrangement (306) in which the clean room device (300) is to be located.

10. The decontamination system according to any of claims 1 to 9, wherein the clean room device (300) comprises a control unit (510) configured for driving the electromagnetic radiation source (302) for emitting the electromagnetic radiation in accordance with a predefined decontaminating procedure.

11. A method of using a clean room device (300) for irradiating parts of a clean room equipment (304) located at a clean room arrangement (306) for decontaminating a surface to be treated of the clean room equipment (304),
wherein the clean room device (300) comprises a movably configured electromagnetic radiation source (302) for irradiating parts of the clean room equipment (304) with electromagnetic radiation for decontaminating a surface to be treated of the clean room equipment (304); wherein the clean room arrangement (306) comprises a clean room lock;
wherein the clean room equipment (304) is configured as clean room clothing to be worn by a person in the clean room lock (306).

12. The method according to claim 11, wherein the clean room device (300) is used
(i) by a person transmitting between an interior (236) and an exterior (238) of a clean room; and/or
(ii) for the microbial decontamination of a surface to be treated of the clean room equipment (304) to be treated.

## Patentansprüche

1. Ein Dekontaminationssystem, wobei das Dekontaminationssystem aufweist:
eine Reinraum Anordnung (306), welche aufweist eine aus der Gruppe bestehend aus einem Reinraum und einer Reinraumschleuse;
eine Reinraumvorrichtung (300), welche an der Reinraum Anordnung (306) angeordnet ist, wobei die Reinraumvorrichtung (300) eine bewegbar konfigurierte elektromagnetische Strahlungsquelle (302) aufweist zum Bestrahlen von Teilen einer Reinraum Ausrüstung (304) mit elektromagnetischer Strahlung zum Dekontaminieren einer zu behandelnden Oberfläche der Reinraum Ausrüstung (304).

2. Das Dekontaminationssystem gemäß Anspruch 1, wobei der Reinraum ein Isolator oder ein Digestorium ist.

3. Das Dekontaminationssystem gemäß Anspruch 1 oder 2, welches zumindest eine weitre elektromagnetische Strahlungsquelle (312) aufweist, welche fest an der Reinraum Anordnung (306) befestigt ist, um elektromagnetische Strahlung auf eine feste Position von der Reinraum Anordnung (306) zu emittieren.

4. Das Dekontaminationssystem gemäß Anspruch 3, wobei die zumindest eine weitere elektromagnetische Strahlungsquelle (312) als eine elektromagnetische Strahlungsquelle (302) konfiguriert ist, welche zumindest eines der folgenden Merkmale (i) und (ii) hat:
(i) die elektromagnetische Strahlungsquelle (302), welche konfiguriert ist zum Emittieren der elektromagnetischen Strahlung mit einer Wellenlänge in einem Bereich, konfiguriert zum Aktivieren eines oder mehrerer Photosensibilisatoren, wobei der eine oder mehrere Photosensibilisatoren reaktive Farbstoffe, und insbesondere reaktive Farbstoffe, sind, welche ausgewählt sind aus der Gruppe bestehend aus Methylenblau, Methyl Methylenblau, Dimethylmethylenblau, Neu Methylenblau, Toluidinblau, Rose Bengal, Acridinorange, und Hypericin;
(ii) die elektromagnetische Strahlungsquelle (302), welche konfiguriert ist zum Emittieren der elektromagnetischen Strahlung, welche emittiert wird eine Wellenlänge in einem Bereich zwischen 400 nm und 800 nm hat, insbesondere in einem Bereich zwischen 580 nm und 650 nm, mehr insbesondere in einem Bereich zwischen 610 nm und 640 nm, noch mehr insbesondere ausschließlich in einem Bereich zwischen 610 nm und 640 nm.

5. Das Dekontaminationssystem gemäß Anspruch 3 oder 4, wobei die zumindest eine weitere elektromagnetische Strahlungsquelle (312) integriert ist in zumindest einem von einer Wand (314), einem Boden (316) und einer Decke (318) der Reinraum Anordnung (306).

6. Das Dekontaminationssystem gemäß einem der Ansprüche 1 bis 5, wobei die Reinraumvorrichtung (300) als eine handgehaltene Vorrichtung (300) konfiguriert ist, wobei die handgehaltene Vorrichtung (300) als integralen Teil davon die elektromagnetische Strahlungsquelle (302) aufweist, insbesondere eine oder mehrere Leuchtdioden (502, 504), zum Bestrahlen von Teilen von der Reinraum Ausrüstung (304), konfiguriert als Reinraum Kleidung, insbesondere ein Reinraumanzug (304), welcher durch eine Person in einer Reinraumschleuse (306) getragen wird, mit elektromagnetischer Strahlung.

7. Das Dekontaminationssystem gemäß Anspruch 6, wobei die handgehaltene Vorrichtung (300) aufweist
einen Griff (308), welcher von einem Anwender greifbar ist und
einen Strahlungskopf (310), welcher mit dem Griff (308) verbunden ist, wobei die elektromagnetische Strahlungsquelle (302) an dem Strahlungskopf (310) angeordnet ist.

8. Das Dekontaminationssystem gemäß einem der Ansprüche 1 bis 5, wobei die Reinraumvorrichtung (400) als ein Scanner (400) konfiguriert ist, insbesondere ein Roboterscanner (400), wobei der Scanner (400) aufweist:
einen Scannerkopf (402), welcher die elektromagnetische Strahlungsquelle (302) als integralen Teil davon aufweist, insbesondere eine oder mehrere Leuchtdioden (502, 504);
eine Antriebseinheit (404), welche konfiguriert ist zum Treiben des Scannerkopfes (402) zum Bewegen entlang einer vordefinierten Trajektorie zum Scannen der Reinraum Ausrüstung (304), um so Teile von der Reinraum Ausrüstung (304) mit elektromagnetischer Strahlung zu bestrahlen.

9. Das Dekontaminationssystem gemäß einem der Ansprüche 1 bis 8, wobei die Reinraumvorrichtung (300) ein Reinraum Interface (508) aufweist, welches konfiguriert ist zum Bereitstellen für eine Kopplung, insbesondere zumindest eines von einer Stromversorgungskopplung zum Versorgen der Reinraumvorrichtung (300) mit Strom und einer Kommunikationskopplung zum Übertragen von Kommunikationsdaten, zwischen der elektromagnetischen Strahlungsquelle (302) und einer Reinraum Anordnung (306), in welcher die Reinraumvorrichtung (300) angeordnet werden soll.

10. Das Dekontaminationssystem gemäß einem der Ansprüche 1 bis 9, wobei die Reinraumvorrichtung (300) eine Steuereinheit (510) aufweist, welche konfiguriert ist zum Treiben der elektromagnetischen Strahlungsquelle (302) zum Emittieren der elektromagnetischen Strahlung in Übereinstimmung mit einer vordefinierten Dekontaminationsprozedur.

11. Ein Verfahren zum Verwenden einer Reinraumvorrichtung (300) zum Bestrahlen von Teilen von einer Reinraum Ausrüstung (304), angeordnet an einer Reinraum Anordnung (306) zum Dekontaminieren einer zu behandelnden Oberfläche von der Reinraum Ausrüstung (304),
wobei die Reinraumvorrichtung (300) eine bewegbar konfigurierte elektromagnetische Strahlungsquelle (302) aufweist zum Bestrahlen von Teilen von der Reinraum Ausrüstung (304) mit elektromagnetischer Strahlung zum Dekontaminieren einer zu behandelnden Oberfläche von der Reinraum Ausrüstung (304), wobei die Reinraum Anordnung (306) eine Reinraumschleuse aufweist;
wobei die Reinraum Ausrüstung (304) konfiguriert ist als Reinraum Kleidung, welche durch eine Person in der Reinraumschleuse (306) getragen wird.

12. Das Verfahren gemäß Anspruch 11, wobei die Reinraumvorrichtung (300) verwendet wird
(i) durch eine Person übertragen zwischen einem Inneren (236) und einen Äußerem (238) von einem Reinraum; und/oder
(ii) für die mikrobiologische Dekontamination von einer zu behandelnden Oberfläche von der zu behandelnden Reinraum Ausrüstung (304).

## Revendications

1. Système de décontamination, le système de décontamination comprenant :
un agencement de salle blanche (306) qui comprend un du groupe constitué d'une salle blanche et d'un sas de salle blanche ;
un dispositif de salle blanche (300) agencé au niveau de l'agencement de salle blanche (306), le dispositif de salle blanche (300) comprenant une source de rayonnement électromagnétique configurée de manière mobile (302) pour irradier des parties d'un équipement de salle blanche (304) avec un rayonnement électromagnétique pour décontaminer une surface à traiter de l'équipement de salle blanche (304).

2. Système de décontamination selon la revendication 1, dans lequel la salle blanche est un isolateur ou une hotte.

3. Système de décontamination selon la revendication 1 ou 2, comprenant au moins une source de rayonnement électromagnétique supplémentaire (312) qui est montée de manière fixe au niveau de l'agencement de salle blanche (306) pour émettre un rayonnement électromagnétique sur une position fixe de l'agencement de salle blanche (306).

4. Système de décontamination selon la revendication 3, dans lequel l'au moins une source de rayonnement électromagnétique supplémentaire (312) est configurée comme une source de rayonnement électromagnétique (302) ayant au moins une des caractéristiques suivantes (i) et (ii) :
(i) la source de rayonnement électromagnétique (302) est configurée pour émettre le rayonnement électromagnétique avec une longueur d'onde dans une plage configurée pour activer un ou plusieurs photosensibilateurs, dans lequel les un ou plusieurs photosensibilateurs sont des colorants réactifs, et particulièrement des colorants réactifs étant sélectionnés à partir du groupe constitué du bleu de méthylène, du bleu de méthylène méthyle, du bleu de diméthylméthylène, du nouveau bleu de méthylène, du bleu de toluidine, du rose Bengale, de l'orange d'acridine et de l'hypéricine ;
(ii) la source de rayonnement électromagnétique (302) est configurée pour émettre le rayonnement électromagnétique qui est émis avec une longueur d'onde dans une plage entre 400 nm et 800 nm, particulièrement dans une plage entre 580 nm et 650 nm, plus particulièrement dans une plage entre 610 nm et 640 nm, encore plus particulièrement exclusivement dans une plage entre 610 nm et 640 nm.

5. Système de décontamination selon la revendication 3 ou 4, dans lequel l'au moins une source de rayonnement électromagnétique supplémentaire (312) est intégrée dans au moins un parmi un mur (314), un plancher (316) et un plafond (318) de l'agencement de salle blanche (306).

6. Système de décontamination selon l'une quelconque des revendications 1 à 5, dans lequel le dispositif de salle blanche (300) est configuré comme un dispositif portatif (300), le dispositif portatif (300) comprenant comme partie intégrante de celui-ci la source de rayonnement électromagnétique (302), particulièrement une ou plusieurs diodes électroluminescentes (502, 504), pour irradier des parties de l'équipement de salle blanche (304) configurée comme un vêtement de salle blanche, particulièrement une tenue de salle blanche (304) à porter par une personne au niveau d'un sas de salle blanche (306), avec un rayonnement électromagnétique.

7. Système de décontamination selon la revendication 6, dans lequel le dispositif portatif (300) comprend une poignée (308) pouvant être saisie par un utilisateur et une tête de rayonnement (310) connectée à la poignée (308), la source de rayonnement électromagnétique (302) étant agencée au niveau de la tête de rayonnement (310).

8. Système de décontamination selon l'une quelconque des revendications 1 à 5, dans lequel le dispositif de salle blanche (400) est configuré comme un scanner (400), particulièrement un scanner robotique (400), le scanner (400) comprenant :
une tête de scanner (402) comprenant comme partie intégrante de celle-ci la source de rayonnement électromagnétique (302), particulièrement une ou plusieurs diodes électroluminescentes (502, 504) ;
une unité d'entraînement (404) configurée pour entraîner la tête de scanner (402) pour se déplacer le long d'une trajectoire prédéfinie pour scanner l'équipement de salle blanche (304) de manière à irradier des parties de l'équipement de salle blanche (304) avec un rayonnement électromagnétique.

9. Système de décontamination selon l'une quelconque des revendications 1 à 8, dans lequel le dispositif de salle blanche (300) comprend une interface de salle blanche (508) configurée pour fournir un couplage, particulièrement au moins un parmi un couplage d'alimentation électrique pour alimenter en énergie le dispositif de salle blanche (300) et un couplage de communication pour transmettre des données de communication, entre la source de rayonnement électromagnétique (302) et un agencement de salle blanche (306) dans lequel le dispositif de salle blanche (300) doit être situé.

10. Système de décontamination selon l'une quelconque des revendications 1 à 9, dans lequel le dispositif de salle blanche (300) comprend une unité de commande (510) configurée pour piloter la source de rayonnement électromagnétique (302) pour émettre le rayonnement électromagnétique conformément à une procédure de décontamination prédéfinie.

11. Procédé d'utilisation d'un dispositif de salle blanche (300) pour irradier des parties d'un équipement de salle blanche (304) situé au niveau d'un agencement de salle blanche (306) pour décontaminer une surface à traiter de l'équipement de salle blanche (304),
dans lequel le dispositif de salle blanche (300) comprenant une source de rayonnement électromagnétique configurée de manière mobile (302) pour irradier des parties de l'équipement de salle blanche (304) avec un rayonnement électromagnétique pour décontaminer une surface à traiter de l'équipement de salle blanche (304) ;
dans lequel l'agencement de salle blanche (306) comprend un sas de salle blanche ;
dans lequel l'équipement de salle blanche (304) est configuré comme un vêtement de salle blanche à porter par une personne dans le sas de salle blanche (306).

12. Procédé selon la revendication 11, dans lequel le dispositif de salle blanche (300) est utilisé
(i) par une personne transmettant entre un intérieur (236) et un extérieur (238) d'une salle blanche ; et/ou
(ii) pour une décontamination d'une surface à traiter de l'équipement de salle blanche (304) à traiter.
